Europäisches Patentamt

European Patent Office

Office européen des brevets

(11) Publication number: **0 323 269 B1**

# EUROPEAN PATENT SPECIFICATION

(45) Date of publication of patent specification: **28.10.92**   (51) Int. Cl.5: **C07C 29/04**, C07C 31/10

(21) Application number: **88312426.5**

(22) Date of filing: **30.12.88**

(54) Process for the hydration of olefins.

(30) Priority: **30.12.87 US 139565**

(43) Date of publication of application:
**05.07.89 Bulletin 89/27**

(45) Publication of the grant of the patent:
**28.10.92 Bulletin 92/44**

(84) Designated Contracting States:
**BE DE FR GB IT NL SE**

(56) References cited:
**EP-A- 0 127 486**     **EP-A- 0 128 064**
**EP-A- 0 210 793**     **DE-A- 2 437 185**
**DE-A- 3 337 301**     **US-A- 4 214 107**

(73) Proprietor: **MOBIL OIL CORPORATION**
**3225 Gallows Road**
**Fairfax, Virginia 22037-0001(US)**

(72) Inventor: **Marler, David O.**
**428 Burd Street**
**Pennington, NJ 08534(US)**
Inventor: **Sorensen, Charles M.**
**3303 Hermitage Road**
**Wilmington, DE 19810(US)**
Inventor: **Varghese, Philip**
**8 Chadwick Dr.**
**Voorhees, NJ 08043(US)**

(74) Representative: **Jones, Alan John et al**
**CARPMAELS & RANSFORD 43 Bloomsbury**
**Square**
**London, WC1A 2RA(GB)**

**Description**

This invention relates to a process for the catalytic hydration of propylene to provide isopropyl alcohol employing zeolite ZSM-35 as catalyst and using relatively low water to propylene mole ratios, i.e., less than about 1. The product isopropyl alcohol is useful, inter alia, as a high octane blending stock for gasoline, as a solvent and as an intermediate for a variety of industrial chemical syntheses.

There is a need for an efficient catalytic process to manufacture alcohols from light olefins thereby augmenting the supply of high octane blending stocks for gasoline. Lower molecular weight alcohols such as isopropyl alcohol (IPA) are in the gasoline boiling range and are known to have a high blending octane number. In addition, by-product propylene from which IPA can be made is usually available at low cost in a petroleum refinery.

The catalytic hydration of olefins to provide alcohols is a well-established art and is of significant commercial importance. Olefin hydration employing zeolite catalyst is known. As disclosed in US-A-4,214,107, monoolefins in the $C_{2-4}$ range, specifically ethylene, propylene, n-butene-1 and cis and trans n-butene-2, are reacted with water at olefin:water mole ratios of from about 0.1:1 to 2:1, preferably from about 0.5:1 to 1.5:1 (equivalent to water:olefin mole ratios of from about 10:1 to about 0.5:1 and preferably from about 2:1 to about 0.67:1) to provide the corresponding alcohol, essentially free of ether and hydrocarbon by-product, employing as olefin hydration catalyst a zeolite having a Constraint Index of 1 to 12, as exemplified by ZSM-5, ZSM-11, ZSM-12, ZSM-35 and ZSM-38. Of the foregoing zeolites, only acidic ZSM-5 is illustrated in a working example.

According to US-A-4,499,313 an olefin is hydrated to the corresponding alcohol in the presence of hydrogen-type mordenite or hydrogen-type zeolite Y each having a silica-alumina molar ratio of 20 to 500. The use of such a catalyst is said to result in higher yields of alcohol than olefin hydration processes which employ conventional solid acid catalysts. Use of the catalyst is said to offer the advantage over ion-exchange type olefin hydration catalysts of not being restricted by the hydration temperature. Reaction conditions employed in the process include a temperature of from 50-300°C, preferably 100-250°C, a pressure of 5 to 200 kg/cm$^2$ to maintain liquid phase or gas-liquid multi-phase conditions and a mole ratio of water to olefin of from 1 to 20. The reaction time can be 20 minutes to 20 hours when operating batchwise and the liquid hourly space velocity (LHSV) is usually 0.1 to 10 in the case of continuous operation.

EP-A-210,793 describes an olefin hydration process employing a medium pore zeolite as hydration catalyst. Specific catalysts mentioned are Theta-1, said to be preferred, ferrierite, ZSM-22, ZSM-23 and NU-10.

By way of realising the foregoing and other objects of the invention, a process for converting propylene to isopropyl alcohol(s) is provided which comprises contacting water with a feed containing at least a substantial amount of propylene in a mole ratio of water to propylene of 0.2 to 0.8 l in the vapour and/or liquid phase under propylene hydration conditions in the presence of zeolite ZSM-35 as propylene hydration catalyst to produce isopropyl alcohol. at a temperature of 50-300°C and a total system pressure of at least 5 atm.(bar).

At the aforestated water:propylene mole ratio ZSM-35, particularly in the hydrogen form, has been found to be far more effective a catalyst for the conversion of propylene to isopropyl alcohol than acidic ZSM-5. Furthermore, ferrierite, a zeolite structurally similar to ZSM-35, has also been observed to be far less catalytically active for propylene hydration than ZSM-35.

The present invention is applicable to the hydration of essentially pure propylene or propylene in admixture with one or more materials which may or may not contain other hydratable olefins. Examples of propylene-containing streams which are useful feeds due to their low cost and ready availability in petroleum refineries include gas plant off-gas containing ethylene and propylene and refinery FCC propane/propylene streams. For example, a typical FCC light olefin stream possesses the following composition:

2

| Typical Refinery FCC Light Olefin Composition | | |
|---|---|---|
| | Wt.% | Mole% |
| Ethane | 3.3 | 5.1 |
| Ethylene | 0.7 | 1.2 |
| Propane | 14.5 | 15.3 |
| Propylene | 42.5 | 46.8 |
| Isobutane | 12.9 | 10.3 |
| n-Butane | 3.3 | 2.6 |
| Butenes | 22.1 | 18.3 |
| Pentanes | 0.7 | 0.4 |

In order to achieve high propylene conversion it is necessary to carry out the hydration at low water to propylene mole ratios, i.e. from 0.2 to 0.8 and preferably from 0.3 to 0.7. A water to propylene mole ratio not exceeding 0.5 has been found to provide an altogether unexpectedly high percentage of propylene conversion to isopropyl alcohol and will often be the mole ratio of choice.

The preferred operating conditions of the propylene hydration process herein are not especially critical and include a temperature of 90 to 250°C and most preferably from 110 to 220°C and a total system pressure of at least 20 atm (bar) and more preferably at least 40 atm (bar).

The use of ZSM-35 in the propylene hydration process of this invention results in high selectivity for isopropyl alcohol, especially at the aforementioned low water to propylene mole ratios, a result contrasting with that obtained using zeolite Beta as hydration catalyst where selectivity shifts to the production of diisopropyl ether at low water to propylene mole ratios.

The propylene hydration process of this invention can be carried out under liquid phase, vapour phase or mixed vapour-liquid phase conditions in batch or continuous manner using a stirred tank reactor or fixed bed flow reactor, e.g., trickle-bed, liquid-up-flow, liquid-down-flow, counter-current, co-current, etc. Reaction times of from 20 minutes to 20 hours when operating in batch and an LHSV of from 0.1 to 10 when operating continuously are suitable. It is generally preferable to recover any unreacted propylene and recycle it to the reactor.

ZSM-35 is described in US-A-4,016,245. Crystals of relatively small average size, e.g., below 0.5 microns and preferably below 0.2 microns, may be advantageously employed herein due to the increased rates of reaction which are generally associated with their use.

In general, the ZSM-35 employed will possess a silica to alumina ratio of at least 10, preferably greater than 20 and more preferably still, greater than 200, e.g., up to 500 and even higher. In place of all or a part of the aluminium present in the framework structure of the zeolite, other trivalent elements can be present such as gallium, iron, boron, etc.

The ZSM-35 for use herein will generally possess an alpha value of at least 1, preferably at least 10 and more preferably at least 100. (The alpha test is described in J. Catalysis, 6 pp. 278-287 (1966) and J. Catalysis, 61, pp. 390-396 (1980).) ZSM-35 of low acidity (alpha values of less than about 200) can be prepared by a variety of techniques including (a) synthesising a zeolite with a high silica/alumina ratio, (b) steaming, (c) steaming followed by dealuminsation and d) substituting aluminium with other species. For example, in the case of steaming, the zeolite can be exposed to steam at elevated temperatures ranging from 500 to 1200°F (260 to 649°C) and preferably from 750 to 1000°F (339 to 538°C). This treatment can be accomplished in an atmosphere of 100% steam or an atmosphere consisting of steam and a gas which is substantially inert to the zeolite. A similar treatment can be accomplished at lower temperatures employing elevated pressure, e.g., at from 350 to 700°F (177 to 371°C) with from 10 to 200 atmospheres (10.14 to 202.76 bar). Aside from or in addition to any of the foregoing procedures, the surface acidity of the zeolite can be eliminated or reduced by treatment with bulky reagents as described in US-A-4,520,221.

In practising the olefin hydration process of the present invention, it can be advantageous to composite the zeolite with a matrix or binder, material which is resistant to the temperature and other conditions employed in the process. Useful matrix materials include both synthetic and naturally-occurring substances, e.g,, inorganic materials such as clay, silica and/or metal oxides. The latter can be either naturally-occurring or can be provided in the form of gelatinous precipitates or gels including mixtures of silica and metal oxides. Naturally-occurring clays which can be composited with the zeolite include those of the montmoril-lonite and kaolin families, which families include the sub-bentonites and the kaolins commonly known as Dixie, McNamee-Georgia and Florida clays or others in which the main mineral constituent is haloysite, kaolinite, dickite, nacrite or anauxite. Such clays can be used in the raw state as originally mined or initially

3

EP 0 323 269 B1

subjected to calcination, acid treatment or chemical modification.

In addition to the foregoing materials, ZSM-35 can be composited with a porous matrix material such as carbon, alumina, silica, titania, zirconia, silica-alumina, silica-magnesia, silica-zirconia, silica-thoria, silica-beryllia and silica-titania, etc, as well as ternary oxide compositions such as silica-alumina-thoria, silica-alumina-zirconia, silica-alumina-magnesia and silica-magnesia-zirconia. The matrix can be in the form of a cogel. The relative proportions of zeolite component and matrix material, on an anhydrous basis, can vary widely with the zeolite content ranging from between 1 to 99 wt%, and more usually in the range of 5 to 90 wt%, of the dry composite.

In some cases, it may be advantageous to provide the ZSM-35 as an extrudate bound with a low acidity refractory oxide binder prepared by forming a homogeneous mixture of ZSM-35, water and a low acidity refractory oxide binder, e.g., silica, which contains at least an extrusion-facilitating amount of the binder in a colloidal state and which is substantially free of added alkali metal base and/or base salt, into an extrudable mass. The mass is extruded and the resulting extrudate dried and calcined.

The original cations associated with ZSM-35 (which may have been synthesised through the agency of pyrrolidine or ethylenediamine) can be replaced by a wide variety of other cations according to techniques well known in the art, e.g., by ion-exchange. Typical replacing cations include hydrogen, ammonium, alkyl ammonium and metal cations, and their mixtures. Metal cations can also be introduced into the zeolite. In the case of metal cations, particular preference is given to metals of Groups IB to VIII of the Periodic Table, including, by way of example, iron, nickel, cobalt, copper, zinc, palladium, calcium, chromium, tungsten, molybdenum, rare earth metals, etc. These metals can also be present in the form of their oxides.

In the following examples, the zeolites are in the acidic (i.e., the hydrogen) form and all percentages are by weight unless otherwise indicated.

EXAMPLE 1
− − − − − − −

This example illustrates the preparation of an alumina-bound ZSM-35 catalyst composition employing pyrrolidine.

Pyrrolidine in an amount of 3.2 weight parts was added to a mixture of 1.38 weight parts 50 weight percent aqueous sodium hydroxide, 1.18 weight parts of hydrated aluminium sulfate ($AL_2O_3(SO_4)_3.14H_2O$), 3.2 weight parts amorphous precipitated silica (PPG Industries HiSil 233) and 7.5 weight parts deionised water. The reaction mixture was then heated to 220°F (104.5°C) and stirred in an autoclave at that temperature for crystallisation. After full crystallinity was achieved the resulting crystals were separated from remaining liquid by filtration, washed with water and dried. Analysis of the crystals confirmed the presence of zeolite ZSM-35.

A portion of the crystals was combined with alumina to form a mixture of 65 weight parts zeolite ZSM-35 and 35 weight parts alumina. Enough water was added to the mixture so that the resulting catalyst could be formed into an extrudate. The catalyst was activated by calcining first in nitrogen at 1000°F (538°C), followed by aqueous exchanges with 1.0 N ammonium nitrate solution and calcining in air at 1000°F (538°C) and 1200°F (649°C).

EXAMPLE 2
− − − − − − −

This example illustrates the preparation of ZSM-35 employing ethylenediamine.

Following preparation of a solution of 101.6g sodium silicate (28.8% $SiO_2$, 8.9% $Na_2O$ and 62.2% $H_2O$), 6.5g NaOH (50% solution) and 59.8g $H_2O$, 30.0g ethylenediamine was added thereto. To this mixture was added a solution comprised of 19.4g $Al_2(SO_4)_3.18H_2O$, 4.5g $H_2SO_4$ and 174g $H_2O$, and the resultant gel was mixed until homogeneous. This gel was composed of the following components in mole ratios:

4

$$\frac{R^+}{R^+ + M^+} \quad 0.82$$

wherein M is sodium and
R is $H_2N(CH_2)_2NH_2$

$$\frac{OH^-}{SiO_2} \quad 0.22$$

$$\frac{H_2O}{OH^-} \quad 152$$

$$\frac{SiO_2}{Al_2O_3} \quad 16.7$$

The gel mixture was maintained at 210°F (99°C) for 62 days, after which time crystallisation was complete. The product crystals were filtered and water washed. Analysis of the crystalline product confirmed the presence of ZSM-35.

EXAMPLE 3

This example compares the catalytic performance of the alumina-bound ZSM-35 catalyst composition of Example 1 with ferrierite (Toyo Soda), also bound with 35 weight percent alumina, for the hydration of light olefin, specifically, propylene. The approximate crystal size of the two zeolites, as estimated, was less than 0.2 microns ($\mu$m) in the case of the ZSM-35 catalyst and from 0.2 to 1.0 microns ($\mu$m) in the case of the ferrierite catalyst.

Two compositions were each employed at two different mole ratios of water:propylene, namely 2:1 and 0.5:1. The other reaction conditions employed were 1000 psig (70 bar), 330°F (166°C) and 0.6 WHSV based on propylene and zeolite. The results are set forth in Table 1 as follows:

## Table 1: Comparison of ZSM-35 and Ferrierite For the Hydration of Propylene

|  | ZSM-35 | | Ferrierite | |
|---|---|---|---|---|
| Mole Ratio Water:Propylene | 2:1 | 0.5:1 | 2:1 | 0.5:1 |
| Water Conversion, % | 25.3 | 46.2 | 0.5 | 3.9 |
| Propylene Conversion, % | 55.1 | 31.0 | 8.7 | 6.2 |
| Alcohol Selectivity, % | 99.5 | 99.3 | 98.7 | 99.6 |

These data demonstrate the clear superiority of ZSM-35 over ferrierite for the catalysis of propylene hydration. In addition these data demonstrate, by means of the comparative runs at water/propylene mole ratio of 2:1, the unexpected benefit of operating with ZSM-35 at unusually low water to propylene mole ratios.

EXAMPLES 4-14

Further comparisons were made between the catalytic performance at 70 bar pressure of alumina-bound ZSM-35 (Examples 4-11) and alumina-bound ferrierite (Examples 12-14), the results confirming the observations and conclusions of Example 3. Examples 4,8,9,10, and 11 are comparative.

The conditions of each reaction (302°F = 150°C; 330°F = 166°C; 379°F = 193°C; 381°F = 194°C) and the results thereof are set forth in Table 2 as follows:

5

## Claims

**Table 2: Comparison of ZSM-35 and Ferrierite in the Hydration of Propylene**

| REACTION CONDITIONS | EXAMPLE | | | | | | | | | | |
|---|---|---|---|---|---|---|---|---|---|---|---|
| | 4 | 5 | 6 | 7 | 8 | 9 | 10 | 11 | 12 | 13 | 14 |
| Reactor Pressure (psig) | 1000.00 | 1000.00 | 1000.00 | 1000.00 | 1000.00 | 1000.00 | 1000.00 | 1000.00 | 1000.00 | 1000.00 | 1000.00 |
| Average Temperature (°F) | 330.00 | 330.00 | 330.00 | 302.00 | 301.00 | 330.00 | 330.00 | 379.00 | 330.00 | 330.00 | 381.00 |
| Water:Propylene Molar Ratio | 2.00 | 0.50 | 0.50 | 0.50 | 2.10 | 2.00 | 2.70 | 2.00 | 2.00 | 0.50 | 2.00 |
| Time on Stream (hr) | 19.50 | 43.50 | 67.50 | 91.50 | 163.25 | 186.25 | 210.25 | 231.25 | 21.25 | 45.25 | 66.00 |
| Space Velocity (WHSV) | 0.62 | 0.62 | 0.63 | 0.62 | 0.30 | 0.62 | 0.23 | 0.63 | 0.63 | 0.63 | 0.63 |
| Space Velocity (LHSV) | 0.64 | 0.49 | 0.49 | 0.49 | 0.31 | 0.65 | 0.26 | 0.65 | 0.67 | 0.51 | 0.67 |
| **Feed Composition, Wt.%** | | | | | | | | | | | |
| Water | 48.84 | 20.03 | 19.39 | 19.63 | 47.61 | 48.78 | 53.81 | 48.60 | 47.48 | 20.90 | 47.82 |
| Propylene | 51.16 | 79.97 | 80.61 | 80.37 | 52.39 | 51.22 | 46.19 | 51.40 | 52.52 | 79.10 | 52.18 |
| **Product Dist., Wt.%** | | | | | | | | | | | |
| Water | 36.49 | 10.77 | 11.38 | 15.80 | 43.63 | 44.67 | 47.55 | 42.12 | 47.26 | 20.09 | 43.83 |
| Propylene | 23.00 | 55.18 | 58.17 | 69.22 | 42.15 | 38.37 | 27.37 | 37.25 | 47.97 | 74.20 | 43.58 |
| 2-Propanol | 40.31 | 33.82 | 30.25 | 14.98 | 14.23 | 16.84 | 25.00 | 20.27 | 4.71 | 5.70 | 12.46 |
| Hexanes | 0.07 | 0.10 | 0.06 | 0.00 | 0.00 | 0.03 | 0.04 | 0.25 | 0.00 | 0.00 | 0.02 |
| Isopropyl Ether | 0.13 | 0.13 | 0.15 | 0.00 | 0.00 | 0.08 | 0.03 | 0.11 | 0.06 | 0.02 | 0.11 |
| **Reactant Conversions, %** | | | | | | | | | | | |
| Total Conversion | 40.51 | 34.05 | 30.46 | 14.98 | 14.23 | 16.96 | 25.08 | 20.63 | 4.77 | 5.72 | 12.59 |
| Water | 25.27 | 46.23 | 41.32 | 19.49 | 8.36 | 8.41 | 11.63 | 13.35 | 0.47 | 3.90 | 8.35 |
| Propylene | 55.05 | 31.00 | 27.84 | 13.87 | 19.56 | 25.09 | 40.75 | 27.53 | 8.66 | 6.20 | 16.48 |
| **Conversion To Ranges, %** | | | | | | | | | | | |
| To C3's | 40.31 | 33.82 | 30.25 | 14.98 | 14.23 | 16.84 | 25.00 | 20.27 | 4.71 | 5.70 | 12.46 |
| To C6+ | 0.20 | 0.23 | 0.21 | 0.00 | 0.00 | 0.12 | 0.08 | 0.36 | 0.06 | 0.02 | 0.13 |
| **Mass Balance Closures, %** | | | | | | | | | | | |
| Total Mass Balance | 94.71 | 95.13 | 93.53 | 97.53 | 96.53 | 95.12 | 96.96 | 94.39 | 95.95 | 97.07 | 97.49 |
| Carbon Balance | 95.15 | 94.05 | 92.27 | 96.73 | 96.00 | 93.34 | 94.34 | 95.10 | 93.76 | 95.97 | 97.93 |
| Hydrogen Balance | 94.77 | 94.93 | 93.29 | 97.38 | 96.46 | 94.89 | 96.65 | 94.48 | 95.66 | 96.87 | 97.55 |
| Oxygen Balance | 94.25 | 99.41 | 98.75 | 100.84 | 97.10 | 96.98 | 99.20 | 93.63 | 98.37 | 101.24 | 97.01 |
| **Product Selectivities, %** | | | | | | | | | | | |
| 2-Propanol | 99.51 | 99.32 | 99.31 | 100.00 | 100.00 | 99.31 | 99.69 | 98.25 | 98.65 | 99.63 | 98.98 |
| Hexanes | 0.18 | 0.29 | 0.18 | 0.00 | 0.00 | 0.20 | 0.18 | 1.22 | 1.35 | 0.37 | 0.86 |
| Isopropyl Ether | 0.31 | 0.39 | 0.51 | 0.00 | 0.00 | 0.49 | 0.13 | 0.53 | ---- | ---- | ---- |

1. A process for converting propylene to isopropyl alcohol which comprises contacting water and a feed containing propylene, at a water-to-propylene mole ratio between 0.2 and 0.8, in the vapour and/or liquid phase with a catalyst comprising zeolite ZSM-35 at 50 to 300 °C and a total system pressure of at least 5 atm. (bar).

2. A process according to claim 1 wherein the propylene is admixed with one or more other materials.

3. A process according to any preceding claim wherein the water to propylene ratio is from 0.3 to 0.7.

4. A process according to any preceding claim wherein the temperature is from 90 to 250°C.

5. A process according to any preceding claim wherein the temperature is from 110 to 220°C.

6. A process according to any preceding claim wherein the total system pressure is at least 20 atm (bar).

7. A process according to any preceding claim wherein the total system pressure is at least 40 atm (bar).

8. A process according to any preceding claim wherein the catalyst comprises a composite of the zeolite with a silica and/or alumina binder.

9. A process according to any preceding claim wherein the framework silica to alumina ratio of the ZSM-35 is at least 10.

10. A process according to any preceding claim wherein unreacted propylene is recycled.

11. A process according to any preceding claim wherein the zeolite is bound with at least an extrusion-facilitating amount of a low acidity refractory oxide binder, provided in a colloidal state, which is substantially free of added alkali metal base and/or basic salt.

12. A process according to any preceding claim wherein the product isopropyl alcohol is substantially free of diisopropyl ether and/or propylene oligomers.

13. A process according to any preceding claim wherein the zeolite is in the hydrogen form.

**Patentansprüche**

1. Verfahren zur Umwandlung von Propylen in Isopropylalkohol, das den Kontakt von Wasser und einer Propylen enthaltenden Beschickung bei einem Molverhältnis von Wasser zu Propylen zwischen 0,2 und 0,8 in der Dampf-und/oder Flüssigkeitsphase mit einem Katalysator, der Zeolith ZSM-35 umfaßt, bei 50 bis 300°C und einem Gesamtdruck des Systems von mindestens 5 atm (bar) umfaßt.

2. Verfahren nach Anspruch 1, worin das Propylen mit einem oder mehreren anderen Materialien vermischt ist.

3. Verfahren nach einem der vorstehenden Ansprüche, worin das Verhältnis von Wasser zu Propylen von 0,3 bis 0,7 beträgt.

4. Verfahren nach einem der vorstehenden Ansprüche, worin die Temperatur von 90 bis 250°C beträgt.

5. Verfahren nach einem der vorstehenden Ansprüche, worin die Temperatur von 110 bis 220°C beträgt.

6. Verfahren nach einem der vorstehenden Ansprüche, worin der Gesamtdruck des Systems mindestens 20 atm (bar) beträgt.

7. Verfahren nach einem der vorstehenden Ansprüche, worin der Gesamtdruck des Systems mindestens 40 atm (bar) beträgt.

8. Verfahren nach einem der vorstehenden Ansprüche, worin der Katalysator ein Verbundmaterial des Zeolith mit einem Siliciumdioxid- und/oder Aluminiumoxidbindemittel umfaßt.

9. Verfahren nach einem der vorstehenden Ansprüche, worin das Siliciumdioxid/Aluminiumoxid-Gitterverhältnis des ZSM-35 mindestens 10 beträgt.

**10.** Verfahren nach einem der vorstehenden Ansprüche, worin das nicht reagierte Propylen rezirkuliert wird.

**11.** Verfahren nach einem der vorstehenden Ansprüche, worin der Zeolith mit zumindest einer die Extrusion erleichternden Menge eines feuerfesten Oxidbindemittels mit geringer Acidität gebunden ist, das im kolloidalen Zustand vorliegt und im wesentlichen frei von einer zugesetzten Alkalimetallbase und/oder einem basischen Salz ist.

**12.** Verfahren nach einem der vorstehenden Ansprüche, worin das Produkt Isopropylalkohol im wesentlicen frei von Diisopropylether und/oder Propylenoligomeren ist.

**13.** Verfahren nach einem der vorstehenden Ansprüche, worin der Zeolith in der Wasserstofform vorliegt.

## Revendications

**1.** Un procédé de conversion du propylène en alcool isopropylique qui comprend la mise en contact d'eau et d'une charge contenant du propylène, avec un rapport molaire eau/propylène compris entre 0,2 et 0,8, en phase vapeur et/ou liquide, avec un catalyseur comprenant une zéolite ZSM-35, à une température comprise entre 50 et 300°C et une pression totale du système d'au moins 5 atmosphères (bars).

**2.** Un procédé selon la revendication 1, dans lequel le propylène est mélangé avec un ou plusieurs autres matériaux.

**3.** Un procédé selon l'une quelconque des revendications précédentes, dans lequel le rapport molaire eau/propylène est de 0,3 à 0,7.

**4.** Un procédé selon l'une quelconque des revendications précédentes, dans lequel la température est comprise entre 90 et 250°C.

**5.** Un procédé selon l'une quelconque des revendications précédentes, dans lequel la température est comprise entre 110 et 220°C.

**6.** Un procédé selon l'une quelconque des revendications précédentes, dans lequel la pression totale du système est d'au moins 20 atmosphères (bars).

**7.** Un procédé selon l'une quelconque des revendications précédentes, dans lequel la pression totale du système est d'au moins 40 atmosphères (bars).

**8.** Un procédé selon l'une quelconque des revendications précédentes, dans lequel le catalyseur comprend un composite du zéolite avec un liant de silice et/ou d'alumine.

**9.** Un procédé selon l'une quelconque des revendications précédentes, dans lequel le rapport silice/alumine de la structure réticulaire de la ZSM-35 est d'au moins 10.

**10.** Un procédé selon l'une quelconque des revendications précédentes, dans lequel le propylène n'ayant pas réagi est recyclé.

**11.** Un procédé selon l'une quelconque des revendications précédentes, dans lequel la zéolite est liée avec au moins une quantité de liant à base d'oxyde réfractaire de faible acidité facilitant l'extrusion, fournie dans un état colloïdal, et qui est substantiellement exempte de base de métal alcalin ajouté et/ou de sel basique.

**12.** Un procédé selon l'une quelconque des revendications précédentes, dans lequel l'alcool isopropylique produit est substantiellement exempt de diisopropyléther et/ou de propylène oligomère.

**13.** Un procédé selon l'une quelconque des revendications précédentes, dans lequel la zéolite est sous sa

8

forme protonée.